(19) Europäisches Patentamt
European Patent Office
Office européen des brevets

(11) EP 4 525 087 A1

(12) **EUROPEAN PATENT APPLICATION**

(43) Date of publication:
**19.03.2025 Bulletin 2025/12**

(21) Application number: **24179763.8**

(22) Date of filing: **04.06.2024**

(51) International Patent Classification (IPC):
***H01M 4/525*** *(2010.01)* ***H01M 10/0567*** *(2010.01)*

(52) Cooperative Patent Classification (CPC):
**H01M 4/525; H01M 10/0567;** Y02E 60/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.06.2023 KR 20230072445**

(71) Applicant: **Samsung SDI Co., Ltd.**
**Yongin-si, Gyeonggi-do 17084 (KR)**

(72) Inventors:
- **Park, Hongryeol**
  **17084 Yongin-si, Gyeonggi-do (KR)**
- **Choi, Hyunbong**
  **17084 Yongin-si, Gyeonggi-do (KR)**
- **Park, Injun**
  **17084 Yongin-si, Gyeonggi-do (KR)**
- **Park, Sangwoo**
  **17084 Yongin-si, Gyeonggi-do (KR)**
- **Sohee, Kim**
  **17084 Yongin-si, Gyeonggi-do (KR)**
- **Yang, Yeji**
  **17084 Yongin-si, Gyeonggi-do (KR)**
- **Kim, Sanghoon**
  **17084 Yongin-si, Gyeonggi-do (KR)**

(74) Representative: **Gulde & Partner**
**Patent- und Rechtsanwaltskanzlei mbB**
**Berliner Freiheit 2**
**10785 Berlin (DE)**

# (54) ADDITIVE, ELECTROLYTE FOR RECHARGEABLE LITHIUM BATTERY, AND RECHARGEABLE LITHIUM BATTERY

(57) An additive for use in an electrolyte for a rechargeable lithium battery, the electrolyte including the same, and a rechargeable lithium battery including the electrolyte are provided. The additive includes a compound with two acid anhydride functional groups linked through an aliphatic or aromatic ring.

EP 4 525 087 A1

## Description

## BACKGROUND

### 1. Field

According to one or more embodiments, the present disclosure relates to an additive, an electrolyte for a rechargeable lithium battery including the same, and a rechargeable lithium battery.

### 2. Description of the Related Art

A rechargeable lithium battery may be recharged and has three or more times as high energy density per unit weight as compared to a comparable lead storage battery, nickel-cadmium battery, nickel hydrogen battery, nickel zinc battery and/or the like. It may be also charged at a high rate and thus, is commercially manufactured for a laptop, a cell phone, an electric tool, an electric bike, and/or the like, and researches on improvement of additional energy density have been actively made or pursued.

A rechargeable lithium battery may be realized by injecting an electrolyte into an electrode assembly that includes a positive electrode (including a positive electrode active material) and a negative electrode (including a negative electrode active material).

One of the recent developmental advances in technology for a rechargeable lithium battery is to improve (or provide suitable) high-temperature charge/discharge and storage characteristics and, also, improve (or provide suitable) room-temperature charge/discharge characteristics. In general, rechargeable lithium batteries may experience an increase in electrical resistance and/or a decrease in cycle-life after being repeatedly charged and discharged at room temperature. These undesirable changes may become more severe if utilizing a cobalt-free positive electrode active material and/or storing the rechargeable lithium battery at relatively high temperatures.

## SUMMARY

The invention is defined by the appended claims. The description that follows is subjected to this limitation. Any disclosure lying outside the scope of said claims is only intended for illustrative as well as comparative purposes. One or more aspects are directed toward an electrolyte additive for a rechargeable lithium battery that improves cycle-life characteristics while suppressing or reducing an increase in resistance of the rechargeable lithium battery at room temperature and/or high temperature.

Additional aspects will be set forth in part in the description which follows and, in part, will be apparent from the description, or may be learned by practice of the presented embodiments of the disclosure.

The additive according to some embodiments may form a stable solid electrolyte interface (SEI) film on the surface of the negative electrode, thereby suppressing or reducing an increase in resistance during operation of a rechargeable lithium battery at room temperature and/or high temperature and improving cycle-life characteristics.

## BRIEF DESCRIPTION OF THE DRAWINGS

FIGs. 1-3 are schematic views showing rechargeable lithium batteries according to some embodiments.

## DETAILED DESCRIPTION

Hereinafter, a rechargeable lithium battery according to some embodiments will be described in more detail, with reference to the attached drawings, so that those of ordinary skill in the art can easily implement them. Examples of which are illustrated in the accompanying drawings, wherein like reference numerals refer to like elements throughout, and duplicative descriptions thereof may not be provided. Accordingly, the embodiments are described, by referring to the drawings, to explain aspects of the present description. However, these embodiments are merely examples, and the present disclosure is not limited thereto. Rather the present disclosure may be embodied in many different forms and is defined by the scope of claims.

The terminology utilized herein is utilized to describe embodiments only, and is not intended to limit the present disclosure. The singular expressions "a," "an," and "the" include the plural expressions, including "at least one," unless the context clearly dictates otherwise.

As utilized herein, "combination thereof" refers to a mixture, laminate, composite, copolymer, alloy, blend, reaction product, and/or the like of the constituents.

Herein, it should be understood that terms such as "comprises," "comprise," "comprising," "includes," "including,"

"include," "having," "has," and/or "have" are intended to designate the presence of an embodied aspect, number, operation, element, and/or a combination thereof, but it does not preclude the possibility of the presence or addition of one or more other feature, number, operation, element, and/or a combination thereof.

In the drawings, the thickness of layers, films, panels, regions, and/or the like, are exaggerated for clarity wherein like reference numerals designate like elements, and duplicative descriptions thereof may not be provided throughout the specification. It will be understood that if an element such as a layer, film, region, or substrate is referred to as being "on" another element, it can be directly on the other element or intervening elements may also be present. In contrast, if an element is referred to as being "directly on" another element, there are no intervening elements present.

In one or more embodiments, the term "layer" herein includes not only a shape formed on the whole surface if viewed from a plan view, but also a shape formed on a partial surface.

It will be understood that, although the terms "first," "second," "third," and/or the like may be utilized herein to describe one or more suitable elements, components, regions, layers and/or sections, these elements, components, regions, layers and/or sections should not be limited by these terms. These terms are only utilized to distinguish one element, component, region, layer or section from another element, component, region, layer, or section. Thus, a first element, component, region, layer, or section described herein may be termed a second element, component, region, layer or section without departing from the teachings set forth herein.

As utilized herein, the term "and/or" includes any, and all, combinations of one or more of the associated listed items. Expressions such as "at least one of," "one of," and "selected from," if preceding a list of elements, modify the entire list of elements and do not modify the individual elements of the list. For example, the expressions "at least one of a to c," "at least one of a, b or c," and "at least one of a, b and/or c" may indicate only a, only b, only c, both (e.g., simultaneously) a and b, both (e.g., simultaneously) a and c, both (e.g., simultaneously) b and c, all of a, b, and c, or variations thereof.

Spatially relative terms, such as "beneath," "below," "lower," "above," "upper" and/or the like, may be utilized herein to easily describe the relationship between one element or feature and another element or feature. It will be understood that the spatially relative terms are intended to encompass different orientations of a device in utilization or operation in addition to the orientation illustrated in the drawings. For example, if the device in the drawings is turned over, elements described as "below" or "beneath" other elements or features will be oriented "above" the other elements or features. Thus, the example term "below" can encompass both (e.g., simultaneously) the orientations of above and below. The device may be otherwise oriented (rotated 90 degrees or at other orientations), and the spatially relative terms utilized herein may be interpreted accordingly.

The terminology utilized herein is utilized for the purpose of describing particular embodiments only, and is not intended to limit the present disclosure. Unless otherwise defined, all terms (including chemical, technical and scientific terms) utilized herein have the same meaning as commonly understood by one of ordinary skill in the art to which this disclosure pertains. It will be further understood that terms, such as those defined in commonly utilized dictionaries, should be interpreted as having a meaning that is consistent with their meaning in the context of the related art and the present disclosure, and will not be interpreted in an idealized or overly formal sense.

Example embodiments are described herein with reference to cross-sectional views, which are schematic views of idealized embodiments. As such, variations from the shapes of the illustrations as a result, for example, of manufacturing techniques and/or tolerances, are to be expected. Thus, embodiments described herein should not be construed as being limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the drawings are schematic in nature and their shapes are not intended to illustrate the precise shape of a region and are not intended to limit the scope of the present claims.

The term "may" will be understood to refer to "one or more embodiments of the present disclosure," some of which include the described element and some of which exclude that element and/or include an alternate element. Similarly, alternative language such as "or" refers to "one or more embodiments of the present disclosure," each including a corresponding listed item.

In this context, "consisting essentially of" means that any additional components will not materially affect the chemical, physical, optical or electrical properties of the semiconductor film.

Further, in this specification, the phrase "on a plane," or "plan view," means viewing a target portion from the top, and the phrase "on a cross-section" means viewing a cross-section formed by vertically cutting a target portion from the side.

**Definitions**

The term "particle diameter" as utilized herein refers to an average diameter of particles if the particles are spherical, and refers to an average major axis length of particles if the particles are non-spherical. For example, the average particle diameter may be measured by a method well suitable to those skilled in the art, for example, may be

measured by a particle size analyzer, or may be measured by a transmission electron microscopic image or a scanning electron microscopic image. It may be possible to obtain an average particle diameter value by measuring utilizing a dynamic light scattering method, performing data analysis, counting the number of particles for each particle size range, and calculating from this. Unless otherwise defined, the average particle diameter may refer to the diameter ($D_{50}$) of particles having a cumulative volume of 50 volume% in the particle size distribution. If measuring by laser diffraction, more specifically, the particles to be measured are dispersed in a dispersion medium and then introduced into a related art laser diffraction particle size measuring device (e.g., MT 3000 available from Microtrac, Ltd.) utilizing ultrasonic waves at 28 kHz, and after irradiation with an output of 60 W, the average particle diameter (D50) based on 50% of the particle size distribution in the measuring device can be calculated. As utilized herein, if a definition is not otherwise provided, the average particle diameter refers to a diameter ($D_{50}$) of particles having a cumulative volume of 50 volume% in the particle size distribution that is obtained by measuring the size (diameter or length of the major axis) of 20 particles at random in a scanning electron microscopic image.

In present disclosure, "not include a or any 'component'" "exclude a or any 'component'", "'component'-free", and/or the like refers to that the "component" not being added, selected or utilized as a component in the composition/-structure, but the "component" of less than a suitable amount may still be included due to other impurities and/or external factors.

"Metal" is interpreted as a concept including ordinary metals, transition metals and metalloids (semi-metals).

As utilized herein, "substituted" refers to replacement of at least one hydrogen of a compound by a substituent selected from among a halogen atom (F, Cl, Br, or I), a hydroxy group, a C1 to C20 alkoxy group, a nitro group, a cyano group, amine group, an imino group, an azido group, an amidino group, a hydrazino group, a hydrazono group, a carbonyl group, a carbamyl group, a thiol group, an ester group, an ether group, a carboxyl group or a salt thereof, a sulfonic acid group or a salt thereof, a phosphoric acid or a salt thereof, a C1 to C20 alkyl group, a C2 to C20 alkenyl group, a C2 to C20 alkynyl group, a C6 to C20 aryl group, a C3 to C20 cycloalkyl group, a C3 to C20 cycloalkenyl group, a C3 to C20 cycloalkynyl group, a C2 to C20 heterocycloalkyl group, a C2 to C20 heterocycloalkenyl group, a C2 to C20 heterocycloalkynyl group, a C3 to C20 heteroaryl group, and/or a combination thereof.

Herein, for example, C1 to C20 refers to having 1 to 20 carbon atoms.

**Additive**

An electrolyte additive for a rechargeable lithium battery is represented by Chemical Formula 1:

Chemical Formula 1

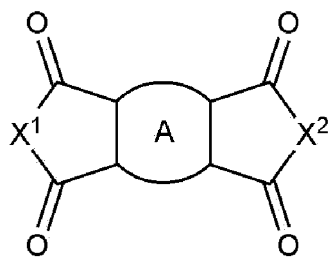

In Chemical Formula 1, A is a substituted or unsubstituted C3 to C10 aliphatic ring, or a substituted or unsubstituted C6 to C20 aromatic ring; and $X^1$ and $X^2$ are each independently O or S.

The additive is a compound with a structure having two acid anhydrides that are linked through an aliphatic or aromatic ring (Chemical Formula 1). Herein, the compound with a structure having two acid anhydrides linked through an aliphatic or aromatic ring (Chemical Formula 1) may be reduced and/or decomposed on the negative electrode surface during operation of a rechargeable lithium battery, e.g., to form a stable solid electrolyte interface (SEI) film. This stable SEI film formed on the negative electrode surface may improve cycle-life characteristics as well as suppress or reduce an increase in resistance during operation of the rechargeable lithium battery at room temperature and/or high temperature.

The description of Chemical Formula 1 representing the additive is as follows.

A may be a cyclohexane ring or a benzene ring.

$X^1$ and $X^2$ may both (e.g., simultaneously) be O.

Representative examples of the additive represented by Chemical Formula 1 may include Chemical Formula 1-1 and 1-2, as follows:

Chemical Formula 1-1

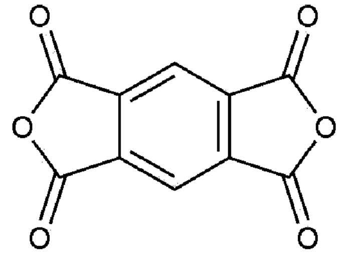

Chemical Formula 1-2

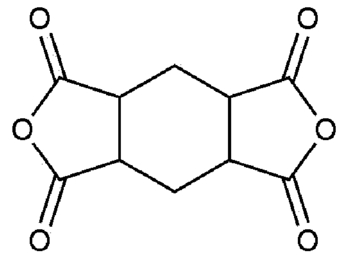

**Electrolyte**

An electrolyte for a rechargeable lithium battery includes a non-aqueous organic solvent; a lithium salt; and an additive represented by Chemical Formula 1.

Chemical Formula 1

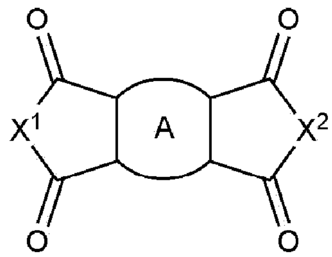

The description of the additive is as described herein.

The electrolyte of some embodiments may form a stable SEI (solid electrolyte interphase) film on the negative electrode surface through the additive and thus improve cycle-life characteristics as well as suppress or reduce an increase in resistance during operation of a rechargeable lithium battery at room temperature and/or a high temperature.

Description of the additive is the same as described in the aforementioned one or more embodiments.

**Content of Additive**

The additive may be included in an amount of 0.05 to 3.0 wt%, 0.5 to 3.0 wt%, 0.05 to 2 wt%, or 0.05 to 1 wt%, based on 100 wt% of the electrolyte. Within the described ranges, the compound with a structure having two acid anhydrides linked through an aliphatic or aromatic ring (Chemical Formula 1) may be reduced and decomposed on the negative electrode surface and thus forms a stable SEI film.

**Non-aqueous Organic Solvent**

The non-aqueous organic solvent serves as a medium for transmitting ions taking part in the electrochemical reaction of a battery. For example, the non-aqueous organic solvent may be a carbonate-based, ester-based, ether-based, ketone-based, or alcohol-based solvent, an aprotic solvent, and/or a combination thereof.

The carbonate-based solvent may include dimethyl carbonate (DMC), diethyl carbonate (DEC), dipropyl

carbonate (DPC), methylpropyl carbonate (MPC), ethylpropyl carbonate (EPC), methylethyl carbonate (MEC), ethylene carbonate (EC), propylene carbonate (PC), butylene carbonate (BC), and/or the like. The ester-based solvent may include methyl acetate, ethyl acetate, n-propyl acetate, dimethylacetate, methylpropionate, ethylpropionate, decanolide, mevalonolactone, valerolactone, caprolactone, and/or the like

The ether-based solvent may include dibutyl ether, tetraglyme, diglyme, dimethoxyethane, 2-methyltetrahydrofuran, 2,5-dimethyltetrahydrofuran, tetrahydrofuran, and/or the like. The ketone-based solvent may include cyclohexanone and/or the like. The alcohol-based solvent may include ethyl alcohol, isopropyl alcohol, and/or the like, and the aprotic solvent may include nitriles such as R-CN (wherein R is a C2 to C20 linear, branched, or cyclic hydrocarbon group, a double bond, an aromatic ring, or an ether group), amides such as dimethylformamide, dioxolanes such as 1,3-dioxolane or 1,4-dioxolane, sulfolanes, and/or the like.

The non-aqueous organic solvent may be utilized alone or as a combination with two or more (e.g., of them). If utilized in combination with two or more, a mixing ratio may be appropriately adjusted according to the desired or suitable battery performance, which is well understood by those skilled in the art.

Additionally, if utilizing a carbonate-based solvent, a cyclic carbonate and a chain carbonate may be mixed and utilized. For example, the cyclic carbonate and the chain carbonate may be mixed at a volume ratio of 1:1 to 1:9.

The non-aqueous organic solvent may further include an aromatic hydrocarbon-based organic solvent. For example, a carbonate-based solvent and an aromatic hydrocarbon-based organic solvent may be mixed and utilized in a volume ratio of 1:1 to 30:1.

The electrolyte may further include vinylethyl carbonate, vinylene carbonate, or an ethylene carbonate-based compound, e.g., to improve battery cycle-life.

Representative examples of the ethylene carbonate-based compound may include fluoroethylene carbonate, difluoroethylene carbonate, chloroethylene carbonate, dichloroethylene carbonate, bromoethylene carbonate, dibromoethylene carbonate, nitroethylene carbonate, and cyanoethylene carbonate.

## Lithium Salt

The lithium salt dissolved in the organic solvent supplies lithium ions in a battery, enables the general operation of a rechargeable lithium battery, and improves transportation of the lithium ions between positive and negative electrodes. Examples of the lithium salt may include at least one (e.g., one or more) selected from among $LiPF_6$, $LiBF_4$, $LiSbF_6$, $LiAsF_6$, $LiClO_4$, $LiAlO_2$, $LiAlCl_4$, $LiPO_2F_2$, LiCl, LiI, $LiN(SO_3C_2F_5)_2$, $Li(FSO_2)_2N$ (lithium bis(fluorosulfonyl)imide, LiFSI), $LiC_4F_9SO_3$, $LiN(C_xF_{2x+1}SO_2)(C_yF_{2y+1}SO_2)$ (wherein x and y are integers of 1 to 20), lithium trifluoromethane sulfonate, lithium tetrafluoroethanesulfonate, lithium difluorobis(oxalato)phosphate (LiDFOB), and lithium bis(oxalato) borate (LiBOB).

A concentration of the lithium salt may be within the range of 0.1 M to 2.0 M. If the concentration of lithium salt is within the described range, the electrolyte may have an appropriate or suitable ionic conductivity and viscosity, and thus excellent or suitable performance may be achieved, e.g., the lithium ions may move effectively.

## Rechargeable Lithium Battery

A rechargeable lithium battery includes a positive electrode; a negative electrode; and the electrolyte, as described herein.

The rechargeable lithium battery includes the electrolyte and thus may improve cycle-life characteristics, as well as, suppress or reduce an increase in resistance at room temperature and/or a high temperature.

## Positive Electrode Active Material

The positive electrode may include a positive electrode active material.

The positive electrode active material may include lithiated intercalation compounds that reversibly intercalate and deintercalate lithium ions. For example, one or more types (kinds) of composite oxides of lithium and a metal selected from cobalt, manganese, nickel, and/or a combination thereof may be utilized.

The composite oxide may be a lithium transition metal composite oxide, and specific examples may include lithium nickel-based oxide, lithium cobalt-based oxide, lithium manganese-based oxide, a lithium iron phosphate-based compound, cobalt-free lithium nickel-manganese-based oxide, and/or a combination thereof.

For example, a compound represented by any of the following chemical formulas may be utilized as the composite oxide: $Li_aA_{1-b}X_bO_{2-c}D_c$ ($0.90 \leq a \leq 1.8$, $0 \leq b \leq 0.5$, $0 \leq c \leq 0.05$); $Li_aMn_{2-b}X_bO_{4-c}D_c$ ($0.90 \leq a \leq 1.8$, $0 \leq b \leq 0.5$, $0 \leq c \leq 0.05$); $Li_aNi_{1-b-c}Co_bX_cO_{2-\alpha}D_\alpha$ ($0.90 \leq a \leq 1.8$, $0 \leq b \leq 0.5$, $0 \leq c \leq 0.5$, $0 < \alpha < 2$); $Li_aNi_{1-b-c}Mn_bX_cO_{2-\alpha}D_\alpha$ ($0.90 \leq a \leq 1.8$, $0 \leq b \leq 0.5$, $0 \leq c \leq 0.5$, $0 < \alpha < 2$); $Li_aNi_bCo_cL^1_dG_eO_2$ ($0.90 \leq a \leq 1.8$, $0 \leq b \leq 0.9$, $0 \leq c \leq 0.5$, $0 \leq d \leq 0.5$, $0 \leq e \leq 0.1$); $Li_aNiG_bO_2$ ($0.90 \leq a \leq 1.8$, $0.001 \leq b \leq 0.1$); $Li_aCoG_bO_2$ ($0.90 \leq a \leq 1.8$, $0.001 \leq b \leq 0.1$); $Li_aMn_{1-b}G_bO_2$ ($0.90 \leq a \leq 1.8$, $0.001 \leq$

$b \leq 0.1$); $Li_aMn_2G_bO_4$ ($0.90 \leq a \leq 1.8$, $0.001 \leq b \leq 0.1$); $Li_aMn_{1-g}G_gPO_4$ ($0.90 \leq a \leq 1.8$, $0 \leq g \leq 0.5$); $Li_{(3-f)}Fe_2(PO_4)_3$ ($0 \leq f \leq 2$); and $Li_aFePO_4$ ($0.90 \leq a \leq 1.8$)

In the described chemical formulas, A may be Ni, Co, Mn, and/or a combination thereof; X may be Al, Ni, Co, Mn, Cr, Fe, Mg, Sr, V, a rare earth element, and/or a combination thereof; D may be O, F, S, P, and/or a combination thereof; G may be Al, Cr, Mn, Fe, Mg, La, Ce, Sr, V, and/or a combination thereof; Q may be Ti, Mo, Mn, and/or a combination thereof; Z may be Cr, V, Fe, Sc, Y, and/or a combination thereof; and $L^1$ may be Mn, Al, and/or a combination thereof.

More specifically, the positive electrode active material may include a lithium nickel-based composite oxide represented by Chemical Formula 11.

Chemical Formula 11 $$Li_{a1}Ni_{x1}M^1_{y1}M^2_{z1}O_{2-b1}X_{b1}$$

In Chemical Formula 11, $0.9 \leq a1 \leq 1.2$, $0.6 \leq x1 \leq 1$, $0 \leq y1 \leq 0.3$, $0 \leq z1 \leq 0.3$, $0.9 \leq x1+y1+z1 \leq 1.1$, and $0 \leq b1 \leq 0.1$; $M^1$ and $M^2$ may each independently be one or more element selected from among Al, B, Ba, Ca, Ce, Co, Cr, Cu, Fe, Mg, Mn, Mo, Nb, Si, Sr, Ti, V, W, and Zr; and X may be one or more element selected from among F, P, and S.

In Chemical Formula 1, $0.7 \leq x1 \leq 1$, $0 \leq y1 \leq 0.2$, and $0 \leq z1 \leq 0.2$; $0.75 \leq x1 \leq 1$, $0 \leq y1 \leq 0.18$, and $0 \leq z1 \leq 0.18$; $0.85 \leq x1 \leq 1$, $0 \leq y1 \leq 0.15$, and $0 \leq z1 \leq 0.15$; or $0.9 \leq x1 \leq 1$, $0 \leq y1 \leq 0.1$, and $0 \leq z1 \leq 0.1$.

For example, the positive electrode active material may include a lithium nickel-based composite oxide represented by Chemical Formula 12. The compound represented by Chemical Formula 12 may be a lithium nickel cobalt-based complex oxide.

Chemical Formula 12 $$Li_{a2}Ni_{x2}Co_{y2}M^3_{z2}O_{2-b2}X_{b2}$$

In Chemical Formula 12, $0.9 \leq a2 \leq 1.8$, $0.6 \leq x2 < 1$, $0 < y2 \leq 0.3$, $0 \leq z2 \leq 0.3$, $0.9 \leq x2+y2+z2 \leq 1.1$, and $0 \leq b2 \leq 0.1$, $M^3$ may be one or more element selected from among Al, B, Ba, Ca, Ce, Cr, Fe, Mg, Mn, Mo, Nb, Si, Sr, Ti, V, W, and Zr, and X is one or more element selected from among F, P, and S.

In Chemical Formula 12, $0.7 \leq x2 < 1$, $0 < y2 \leq 0.2$, and $0 \leq z2 \leq 0.2$; $0.75 \leq x2 \leq 0.99$, $0 \leq y2 \leq 0.15$, and $0 \leq z2 \leq 0.15$; $0.85 \leq x2 \leq 0.99$, $0.01 \leq y2 \leq 0.15$, and $0.01 \leq z2 \leq 0.15$; or $0.9 \leq x2 \leq 0.99$, $0.01 \leq y2 \leq 0.1$, and $0.01 \leq z2 \leq 0.1$.

As an example, the positive electrode active material may include a lithium nickel-based composite oxide represented by Chemical Formula 13. The compound of Chemical Formula 13 may be a lithium nickel-cobalt-aluminum oxide or a lithium nickel-cobalt-manganese oxide.

Chemical Formula 13 $$Li_{a3}Ni_{x3}Co_{y3}M^4_{z3}M^5_{w3}O_{2-b3}X_{b3}$$

In Chemical Formula 13, $0.9 \leq a3 \leq 1.8$, $0.6 \leq x3 \leq 0.98$, $0.01 \leq y3 \leq 0.29$, $0.01 \leq z3 \leq 0.29$, $0 \leq w3 \leq 0.29$, $0.9 \leq x3+y3+z3+w3 \leq 1.1$, and $0 \leq b3 \leq 0.1$, $M^4$ may be one or more element selected from among Al, and Mn, $M^5$ may be one or more element selected from B, Ba, Ca, Ce, Cr, Fe, Mg, Mo, Nb, Si, Sr, Ti, V, W, and Zr, and X may be one or more element selected from among F, P, and S.

In Chemical Formula 13, $0.7 \leq x3 \leq 0.98$, $0.01 \leq y3 \leq 0.19$, $0.01 \leq z3 \leq 0.19$, $0 \leq w3 \leq 0.19$, $0.75 \leq x3 \leq 0.98$, $0 \leq y3 \leq 0.16$, and $0 \leq z3 \leq 0.16$; $0.85 \leq x3 \leq 0.98$, $0.01 \leq y3 \leq 0.14$, $0.01 \leq z3 \leq 0.14$, and $0 \leq w3 \leq 0.14$; or $0.9 \leq x3 \leq 0.98$, $0.01 \leq y3 \leq 0.09$, $0.01 \leq z3 \leq 0.09$, and $0 \leq w3 \leq 0.09$.

As an example, the positive electrode active material may include a lithium nickel-based composite oxide represented by Chemical Formula 14. The compound of Formula 14 may be a cobalt-free lithium nickel-manganese oxide.

Chemical Formula 14 $$Li_{a4}Ni_{x4}Mn_{y4}M^6_{z4}O_{2-b4}X_{b4}$$

In Chemical Formula 14, $0.9 \leq a2 \leq 1.8$, $0.6 \leq x4 < 1$, $0 < y4 \leq 0.3$, $0 \leq z4 \leq 0.3$, $0.9 \leq x4+y4+z4 \leq 1.1$, and $0 \leq b4 \leq 0.1$, $M^6$ may be one or more element selected from among Al, B, Ba, Ca, Ce, Cr, Fe, Mg, Mo, Nb, Si, Sr, Ti, V, W, and Zr, and X may be one or more element selected from among F, P, and S.

In Chemical Formula 14, $0.7 \leq x4 < 1$, $0 < y4 \leq 0.2$, and $0 \leq z4 \leq 0.2$; $0.75 \leq x4 \leq 0.99$, $0 \leq y4 \leq 0.15$, and $0 \leq z4 \leq 0.15$; $0.85 \leq x4 \leq 0.99$, $0.01 \leq y4 \leq 0.15$, and $0.01 \leq z4 \leq 0.15$; or $0.9 \leq x4 \leq 0.99$, $0.01 \leq y4 \leq 0.1$, and $0.01 \leq z4 \leq 0.1$.

If the transition metal is eluted from the positive electrode active material and is reduced on the surface of the negative electrode during operation of a rechargeable lithium battery, the structure of the surface of the negative electrode may be weakened. As a result, increased battery resistance and reduced cycle-life may occur, and in the long term, lithium dendrites may be formed, that may cause a short circuit between the positive electrode and negative electrode.

In contrast, recently, in response to cobalt depletion and rising costs, research on so-called "cobalt-free positive electrode active materials" is underway. The cobalt-free positive electrode active material refers to a positive electrode active material with a layered structure composed of nickel, manganese, and/or the like as main components and does not contain cobalt in the positive electrode active material composition. In the rechargeable lithium battery of some

embodiments, one type or kind of cobalt-free lithium nickel-manganese oxide represented by Chemical Formula 14 may be utilized as the cobalt-free positive electrode active material.

However, the cobalt-free positive electrode active material has high structural instability due to the absence of cobalt, so there is a high probability that nickel and/or manganese will be eluted during operation of the rechargeable lithium battery. Additionally, this problem may become more severe at high temperatures.

Nevertheless, if an electrolyte prepared by adding a compound having a structure with two acid anhydrides that are linked through an aliphatic or aromatic ring (Chemical Formula 1) is utilized, even though the cobalt-free positive electrode active material with a layered structure is utilized, the stable SEI film may improve cycle-life characteristics as well as protect the negative electrode surface and suppress or reduce an increase in resistance during operation of a rechargeable lithium battery at room temperature and/or a high temperature.

## Positive Electrode

The positive electrode for a rechargeable lithium battery may include a current collector and a positive electrode active material layer on the current collector. The positive electrode active material layer may include a positive electrode active material and may further include a binder and/or a conductive material.

A content of the positive electrode active material may be 90 wt% to 99.5 wt% based on 100 wt% of the positive electrode active material layer.

In some embodiments, the positive electrode active material layer may further include a binder and a conductive material. At this time, a content of the binder and the conductive material may be 0.5 wt% to 5 wt%, respectively, based on 100 wt% of the positive electrode active material layer.

The binder serves to attach the positive electrode active material particles well to each other and, also, to attach the positive electrode active material well to the current collector. Examples of the binder may include polyvinyl alcohol, carboxymethyl cellulose, hydroxypropyl cellulose, diacetyl cellulose, polyvinylchloride, carboxylated polyvinylchloride, polyvinylfluoride, a polymer including ethylene oxide, polyvinylpyrrolidone, polyurethane, polytetrafluoroethylene, polyvinylidene fluoride, polyethylene, polypropylene, a styrene-butadiene rubber, a (meth)acrylated styrene-butadiene rubber, an epoxy resin, a (meth)acrylic resin, a polyester resin, nylon, and/or the like, as non-limiting examples.

The conductive material may be utilized to impart conductivity (e.g., electrical conductivity) to the electrode. Any material that does not cause chemical change (e.g., does not cause an undesirable chemical change in the rechargeable lithium battery) and conducts electrons can be utilized in the battery. Examples of the conductive material may include a carbon-based material such as natural graphite, artificial graphite, carbon black, acetylene black, ketjen black, a carbon fiber, a carbon nanofiber, and carbon nanotube; a metal-based material containing copper, nickel, aluminum, silver, and/or the like, in a form of a metal powder or a metal fiber; a conductive polymer such as a polyphenylene derivative; or a mixture thereof.

Al may be utilized as the current collector, but the present disclosure is not limited thereto.

## Negative Electrode Active Material

The negative electrode may include a negative electrode active material.

The negative electrode active material may be a material that reversibly intercalates/deintercalates lithium ions, a lithium metal, a lithium metal alloy, a material capable of doping and dedoping lithium, or a transition metal oxide.

The material that reversibly intercalates/deintercalates lithium ions may include a carbon-based negative electrode active material, for example, crystalline carbon, amorphous carbon, and/or a combination thereof. The crystalline carbon may be graphite such as non-shaped, sheet-shaped, flake-shaped, sphere-shaped, or fiber-shaped natural graphite or artificial graphite. The amorphous carbon may be a soft carbon, a hard carbon, a mesophase pitch carbonization product, calcined coke, and/or the like.

The lithium metal alloy may include lithium and a metal selected from among Na, K, Rb, Cs, Fr, Be, Mg, Ca, Sr, Si, Sb, Pb, In, Zn, Ba, Ra, Ge, Al, and Sn.

The material capable of doping/dedoping lithium may be a Si-based negative electrode active material or a Sn-based negative electrode active material. The Si-based negative electrode active material may include silicon, a silicon-carbon composite, $SiO_x$ ($0 < x \leq 2$), a Si-Q alloy (where Q is selected from among an alkali metal, an alkaline-earth metal, a Group 13 element, a Group 14 element (excluding Si), a Group 15 element, a Group 16 element, a transition metal, a rare earth element, and/or a combination thereof, for example Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Tl, Ge, P, As, Sb, Bi, S, Se, Te, Po, and/or a combination thereof). The Sn-based negative electrode active material may include Sn, $SnO_2$, $SnO_x$ ($0 < x < 2$), a Sn-based alloy, and/or a combination thereof.

The silicon-carbon composite may be a composite of silicon and amorphous carbon. An average particle diameter ($D_{50}$) of the silicon-carbon composite particle may be for example 0.5 micrometer ($\mu$m) to 20 $\mu$m. According to

some embodiments, the silicon-carbon composite may be in a form of silicon particles and amorphous carbon coated on the surface of the silicon particles. For example, the silicon-carbon composite may include a secondary particle (core) in which primary silicon particles are assembled, and an amorphous carbon coating layer (shell) on the surface of the secondary particle. The amorphous carbon may also be between the primary silicon particles, and, for example, the primary silicon particles may be coated with the amorphous carbon. The secondary particle may be (exist) dispersed in an amorphous carbon matrix.

The silicon-carbon composite may further include crystalline carbon. For example, the silicon-carbon composite may include a core including crystalline carbon and silicon particles and an amorphous carbon coating layer on a surface of the core. The crystalline carbon may be artificial graphite, natural graphite, and/or a combination thereof. The amorphous carbon may include soft carbon or hard carbon, a mesophase pitch carbonized product, and calcined coke.

If the silicon-carbon composite includes silicon and amorphous carbon, a content of silicon may be 10 wt% to 50 wt%, and a content of amorphous carbon may be 50 wt% to 90 wt% based on 100 wt% of the silicon-carbon composite. In one or more embodiments, if the composite includes silicon, amorphous carbon, and crystalline carbon, a content of silicon may be 10 wt% to 50 wt%, and a content of crystalline carbon may be 10 wt% to 70 wt%, and a content of amorphous carbon may be 20 wt% to 40 wt% based on 100 wt% of the silicon-carbon composite.

Additionally, a thickness of the amorphous carbon coating layer may be 5 nanometer (nm) to 100 nm. An average particle diameter ($D_{50}$) of the silicon particles (primary particles) may be 10 nm to 1 $\mu$m, or 10 nm to 200 nm. The silicon particles may exist as silicon alone, in the form of a silicon alloy, or in an oxidized form. The oxidized form of silicon may be represented by $SiO_x$ ($0<x\leq2$). In some embodiments, an atomic content ratio of Si:O, which indicates a degree of oxidation, may be 99:1 to 33:67. In the present specification, As utilized herein, if a definition is not otherwise provided, an average particle diameter (D50) indicates a particle where an accumulated volume is 50 volume% in a particle distribution.

The Si-based negative electrode active material or the Sn-based negative electrode active material may be utilized in combination with a carbon-based negative electrode active material. If utilizing a mixture of Si-based negative electrode active material or Sn-based negative electrode active material and carbon-based negative electrode active material, a mixing ratio may be 1:99 to 90:10 by weight.

**Negative Electrode**

A negative electrode for a rechargeable lithium battery includes a current collector and a negative electrode active material layer on the current collector. The negative electrode active material layer includes a negative electrode active material and may further include a binder and/or a conductive material.

A content of the negative electrode active material may be 95 wt% to 99.5 wt% based on 100 wt% of the negative electrode active material layer. A content of the binder may be 0.5 wt% to 5 wt% based on 100 wt% of the negative electrode active material layer. A content of the binder may be 0.5 wt% to 5 wt% based on 100 wt% of the negative electrode active material layer. For example, the negative electrode active material layer may include 90 wt% to 99 wt% of the negative electrode active material, 0.5 wt% to 5 wt% of the binder, and 0.5 wt% to 5 wt% of the conductive material.

The binder may serve to attach the negative electrode active material particles well to each other and, also, to attach the negative electrode active material well to the current collector. The binder may include a non-aqueous binder, an aqueous binder, a dry binder, and/or a combination thereof.

The non-aqueous binder may include polyvinyl chloride, carboxylated polyvinyl chloride, polyvinyl fluoride, an ethylene propylene copolymer, polystyrene, polyurethane, polytetrafluoroethylene, polyvinylidene fluoride, polyethylene, polypropylene, polyamideimide, polyimide, and/or a combination thereof.

The aqueous binder may be selected from among a styrene-butadiene rubber, a (meth)acrylated styrene-butadiene rubber, a (meth)acrylonitrile-butadiene rubber, (meth)acrylic rubber, a butyl rubber, a fluoro rubber, polyethylene oxide, polyvinylpyrrolidone, polyepichlorohydrine, polyphosphazene, poly(meth)acrylonitrile, an ethylene propylene diene copolymer, polyvinylpyridine, chlorosulfonated polyethylene, latex, a polyester resin, a (meth)acrylic resin, a phenol resin, an epoxy resin, polyvinyl alcohol, and/or a combination thereof.

If an aqueous binder is utilized as the negative electrode binder, it may further include a cellulose-based compound capable of imparting viscosity. The cellulose-based compound includes one or more of carboxylmethyl cellulose, hydroxypropylmethyl cellulose, methyl cellulose, or alkali metal salts thereof. The alkali metal may be Na, K, and/or Li.

The dry binder may be a polymer material capable of being fiberized, and may be, for example, polytetrafluoroethylene, polyvinylidene fluoride, polyvinylidene fluoride-hexafluoropropylene copolymer, polyethylene oxide, and/or a combination thereof.

The conductive material is included to provide electrode conductivity, and any electrically conductive material may be utilized as a conductive material unless it causes a chemical change. Examples of the conductive material may be a carbon-based material such as natural graphite, artificial graphite, carbon black, acetylene black, ketjen black, a carbon fiber, a carbon nanofiber, a carbon nanotube, and/or the like; a metal-based material such as copper, nickel, aluminum

silver, and/or the like in a form of a metal powder or a metal fiber; a conductive polymer such as a polyphenylene derivative; or a mixture thereof.

The negative electrode current collector may include one selected from among a copper foil, a nickel foil, a stainless steel foil, a titanium foil, a nickel foam, a copper foam, a polymer substrate coated with a conductive metal, and/or a combination thereof, but the present disclosure is not limited thereto.

**Separator**

Depending on the type or kind of the rechargeable lithium battery, a separator may be present between the positive electrode and the negative electrode. The separator may include polyethylene, polypropylene, polyvinylidene fluoride, or a multilayer film of two or more layers thereof, and a mixed multilayer film such as a polyethylene/polypropylene two-layer separator, polyethylene/polypropylene/polyethylene three-layer separator, polypropylene/polyethylene/polypropylene three-layer separator, and/or the like.

The separator may include a porous substrate and a coating layer including an organic material, an inorganic material, and/or a combination thereof on one or both (e.g., simultaneously) surfaces (e.g., opposite surfaces) of the porous substrate.

The porous substrate may be a polymer film formed of any one selected polymer polyolefin such as polyethylene and polypropylene, polyester such as polyethylene terephthalate and polybutylene terephthalate, polyacetal, polyamide, polyimide, polycarbonate, polyether ketone, polyarylether ketone, polyether ketone, polyetherimide, polyamideimide, polybenzimidazole, polyethersulfone, polyphenylene oxide, a cyclic olefin copolymer, polyphenylene sulfide, polyethylene naphthalate, a glass fiber, TEFLON, and polytetrafluoroethylene, or a copolymer or mixture of two or more thereof.

The porous substrate may have a thickness of 1 μm to 40 μm, for example, 1 μm to 30 μm, 1 μm to 20 μm, 5 μm to 15 μm, or 10 μm to 15 μm.

The organic material may include a (meth)acryl-based copolymer including a first structural unit derived from (meth)acrylamide, and a second structural unit including at least one of a structural unit derived from (meth)acrylic acid or (meth)acrylate, and a structural unit derived from (meth)acrylamidosulfonic acid or a salt thereof.

The inorganic material may include inorganic particles selected from among $Al_2O_3$, $SiO_2$, $TiO_2$, $SnO_2$, $CeO_2$, MgO, NiO, CaO, GaO, ZnO, $ZrO_2$, $Y_2O_3$, $SrTiO_3$, $BaTiO_3$, $Mg(OH)_2$, boehmite, and/or a combination thereof, but the present disclosure is not limited thereto. An average particle diameter ($D_{50}$) of the inorganic particles may be 1 nm to 2000 nm, for example, 100 nm to 1000 nm, or 100 nm to 700 nm.

The organic material and the inorganic material may be mixed in one coating layer, or a coating layer including an organic material and a coating layer including an inorganic material may be stacked.

The thickness of the coating layer may be 0.5 μm to 20 μm, for example, 1 μm to 10 μm, or 1 μm to 5 μm.

**Rechargeable Lithium Battery**

The rechargeable lithium battery may be classified into cylindrical, prismatic, pouch, or coin-type or kind batteries, and/or the like depending on their shape. FIGs. 1 to 3 are schematic views showing rechargeable lithium batteries according to some embodiments. Referring to FIGs. 1 to 3, the rechargeable lithium battery 100 includes an electrode assembly 40 with a separator 30 interposed between the positive electrode 10 and the negative electrode 20, and a case 50 in which the electrode assembly 40 is housed. The positive electrode 10, negative electrode 20, and separator 30 may be impregnated with an electrolyte. The rechargeable lithium battery 100 may include a sealing member 60 that seals the case 50 as shown in FIG. 1, and as shown in FIG. 3, it may further include electrode tabs 70 that serve as an electrical path for guiding the current generated in the electrode assembly 40 to the outside.

Numerical ranges disclosed herein include and are intended to disclose all subsumed sub-ranges of the same numerical precision. For example, a range of "1.0 to 10.0" includes all subranges having a minimum value equal to or greater than 1.0 and a maximum value equal to or less than 10.0, such as, for example, 2.4 to 7.6. Applicant therefore reserves the right to amend this specification, including the claims, to expressly recite any sub-range subsumed within the ranges expressly recited herein.

Here, the listing of the process in a particular order should not necessarily means that the invention or claims require that particular order. That is, the general rule that unless the steps, tasks, or acts of a process (e.g., a method) actually recite an order, the steps, tasks, or acts should not be construed to require one.

Hereinafter, examples and comparative examples of the present disclosure will be described. The following example is only an example of the present disclosure, and the present disclosure is not limited to the following examples.

## EXAMPLES

### Manufacture of Electrolyte and Rechargeable Lithium Battery Cells

### Example 1

#### (1) Preparation of Electrolyte

An electrolyte was prepared by mixing ethylene carbonate (EC), ethylmethyl carbonate (EMC), and dimethyl carbonate (DMC) in a volume ratio of 2:4:4, dissolving 1.5 M $LiPF_6$ in the non-aqueous organic solvent, and adding 0.05 wt% of the additive represented by Chemical Formula 1-1 (product name: SKU 412287-100G, manufacturer: Sigma-Aldrich).

Chemical Formula 1-1

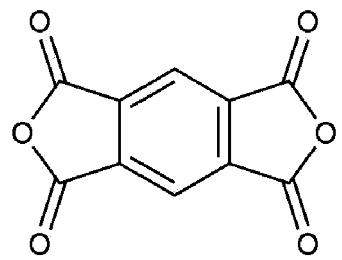

In the composition of the electrolyte, a content of the additive, "wt%" is based on 100 wt% of the total electrolyte (lithium salt + non-aqueous organic solvent + additive).

#### (2) Manufacture of Rechargeable Lithium Battery Cells

$LiNi_{0.75}Mn_{0.23}Al_{0.02}O_2$ as a positive electrode active material, polyvinylidene fluoride as a binder, and acetylene black as a conductive material were mixed respectively in a weight ratio of 96:3:1, and then, dispersed in N-methyl pyrrolidone to prepare positive electrode active material slurry.

The positive electrode active material slurry was coated on a 15 micrometer (μm)-thick Al foil, dried at 100 °C, and pressed to manufacture a positive electrode.

A mixture of artificial graphite and a Si-C composite in a weight ratio of 93:7 was prepared as a negative electrode active material, and the negative electrode active material, a styrene-butadiene rubber binder, and carboxylmethyl cellulose in a weight ratio of 98:1:1 were dispersed in distilled water to prepare a negative electrode active material slurry.

The Si-C composite included a core including artificial graphite and silicon particles and carbonyl pitch coated on the surface of the core.

The negative electrode active material slurry was coated on a 10 μm-thick Cu foil, dried at 100 °C, and pressed to manufacture a negative electrode.

An electrode assembly was manufactured by assembling the positive electrode, the negative electrode, and a separator made of polyethylene with a thickness of 10 μm, and the electrolyte was injected, respectively, to manufacture rechargeable lithium battery cells.

### Example 2

An electrolyte and a rechargeable lithium battery cell were manufactured in substantially the same manner as in Example 1 except that the additive represented by Chemical Formula 1-1 was added in an amount of 0.1 wt%.

### Example 3

An electrolyte and a rechargeable lithium battery cell were manufactured in substantially the same manner as in Example 1 except that the additive represented by Chemical Formula 1-1 was added in an amount of 0.5 wt%.

### Example 4

An electrolyte and a rechargeable lithium battery cell were manufactured in substantially the same manner as in

Example 1 except that the additive represented by Chemical Formula 1-1 was added in an amount of 1.0 wt%.

**Example 5**

An electrolyte and a rechargeable lithium battery cell were manufactured in substantially the same manner as in Example 1 except that the additive represented by Chemical Formula 1-1 was added in an amount of 3.0 wt%.

**Example 6**

An electrolyte and a rechargeable lithium battery cell were manufactured in substantially the same manner as in Example 1 except that the additive represented by Chemical Formula 1-2 (product name: C2419-25G, manufacturer: TCI Chemical Industry) in an amount of 0.05 wt% was added instead of the additive represented by Chemical Formula 1-1.

Chemical Formula 1-2

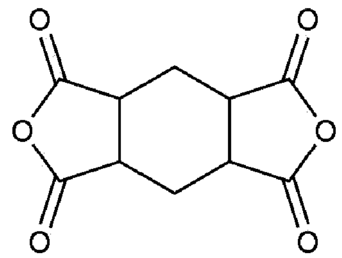

**Example 7**

An electrolyte and a rechargeable lithium battery cell were manufactured in substantially the same manner as in Example 1 except that the additive represented by Chemical Formula 1-2 in an amount of 0.1 wt% was added instead of the additive represented by Chemical Formula 1-1.

**Example 8**

An electrolyte and a rechargeable lithium battery cell were manufactured in substantially the same manner as in Example 1 except that the additive represented by Chemical Formula 1-2 in an amount of 0.5 wt% was added instead of the additive represented by Chemical Formula 1-1.

**Example 9**

An electrolyte and a rechargeable lithium battery cell were manufactured in substantially the same manner as in Example 1 except that the additive represented by Chemical Formula 1-2 in an amount of 1.0 wt% was added instead of the additive represented by Chemical Formula 1-1.

**Example 10**

An electrolyte and a rechargeable lithium battery cell were manufactured in substantially the same manner as in Example 1 except that the additive represented by Chemical Formula 1-2 in an amount of 3.0 wt% was added instead of the additive represented by Chemical Formula 1-1.

**Example 11**

An electrolyte and a rechargeable lithium battery cell were manufactured in substantially the same manner as in Example 1 except that $LiNi_{0.9}Co_{0.05}Al_{0.05}O_2$ was utilized instead of $LiNi_{0.75}Mn_{0.23}Al_{0.02}O_2$ as a positive electrode active material.

**Comparative Example 1 (Ref.)**

An electrolyte and a rechargeable lithium battery cell were manufactured in substantially the same manner as in Example 1 except that no additive was added (e.g., at all).

**Comparative Example 2**

An electrolyte and a rechargeable lithium battery cell were manufactured in substantially the same manner as in Example 1 except that the additive represented by Chemical Formula A in an amount of 0.5 wt% was added instead of the additive represented by Chemical Formula 1-1 (Synthesis Example 1).

Chemical Formula A

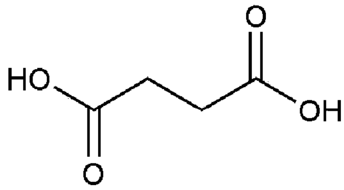

**Evaluation Examples**

The rechargeable lithium battery cells manufactured by respectively utilizing the electrolytes according to Examples 1 to 11 and Comparative Examples 1 and 2 were evaluated as follows, and the results are shown in Tables 1 to 3.

**Evaluation 1: Evaluation of Room-temperature Charge/discharge Cycle Characteristics**

The rechargeable lithium battery cells according to Examples 1 to 11 and Comparative Examples 1 and 2 were charged and discharged under the following conditions to evaluate cycle characteristics, and the results are shown in Table 1.

The cells were 800 cycles charged and discharged under 0.33 C charge (CC/CV, 4.45 V, 0.025 C Cut-off) /1.0 C discharge (CC, 2.5 V Cut-off) conditions at 25 °C to measure capacity retention and a change in direct current internal resistance (DC-IR).

The capacity retention was calculated according to Equation 1, and the DC internal resistance change rate was calculated according to Equation 2 based on a voltage change while discharged by applying a current of SOC 50 C (a current equal to 50% of charge capacity based on 100% of the entire charge capacity of the battery cell), for 30 seconds.

Capacity retention rate = (discharge capacity after 800 cycles / discharge capacity after 1 cycle) * 100

DC internal resistance change rate = {((DC-IR after 800 cycles - DC-IR after 1 cycle)/(DC-IR after 1 cycle)} * 100

Table 1

| | Capacity retention rate @ 25 °C, 800th cycle (%) | Initial resistance @ 25°C (mΩ) | DC-IR after 800th cycle @ 25 °C (mΩ) | DC-IR change rate after 800th cycle @ 25 °C (%) |
|---|---|---|---|---|
| Example 1 | 76.4 | 21.31 | 28.15 | 132.1 |
| Example 2 | 80.8 | 21.98 | 28.22 | 128.4 |
| Example 3 | 88.4 | 22.32 | 27.19 | 121.8 |
| Example 4 | 87.9 | 22.62 | 27.73 | 122.6 |
| Example 5 | 80.2 | 23.34 | 28.99 | 124.2 |
| Example 6 | 71.8 | 21.15 | 28.43 | 134.4 |
| Example 7 | 75.7 | 21.50 | 28.57 | 132.9 |
| Example 8 | 80.2 | 22.42 | 29.48 | 131.5 |
| Example 9 | 79.9 | 22.51 | 29.94 | 133.0 |
| Example 10 | 74.0 | 22.95 | 30.91 | 134.7 |

(continued)

| | Capacity retention rate @ 25 °C, 800th cycle (%) | Initial resistance @ 25°C (mΩ) | DC-IR after 800th cycle @ 25 °C (mΩ) | DC-IR change rate after 800th cycle @ 25 °C (%) |
|---|---|---|---|---|
| Example 11 | 88.5 | 19.18 | 23.00 | 119.9 |
| Comparative Example 1 | 55.4 | 20.97 | 30.76 | 146.7 |
| Comparative Example 2 | 61.0 | 22.90 | 31.83 | 139.0 |

Referring to Table 1, if the additive according to some embodiments was utilized at room temperature, not only were cycle-life characteristics improved, but an increase in resistance was suppressed or reduced.

**Evaluation 2: Evaluation of Storage Characteristics at High Temperature Capacity Retention Rate/Capacity Recovery Rate/DC-IR Change Rate**

**(1) Capacity Retention Rate and Capacity Recovery Rate**

Each of the rechargeable lithium battery cells according to Examples 1 to 11 and Comparative Examples 1 and 2 was once charged and discharged at 0.33 C to measure charge and discharge capacity (before the high-temperature storage).

In one or more embodiments, each of the rechargeable lithium battery cells of Examples 1 to 11 and Comparative Examples 1 and 2 was charged to SOC 100% (a state of being charged to 100% of charge capacity based on 100% of the entire charge capacity of the battery cell), stored at 60 °C for 60 days, and then, discharged to 3.0 V under a constant current condition at 0.33 C to measure initial discharge capacity.

The cells were recharged to 4.3 V at a constant current of 0.33 C and cut off at an ending current of 0.02 C and discharged to 3.0 V at the constant current of 0.33C to twice measure discharge capacity. A ratio of first discharge capacity to the initial discharge capacity was calculated as capacity retention (retention capacity), and second discharge capacity was provided as recovery capacity.

**(2) DC-IR Change Rate**

Initial DC resistance (DCIR) of each of the rechargeable lithium battery cells according to Examples 1 to 11 and Comparative Examples 1 and 2 were measured with $\triangle V/\triangle I$ (voltage change/current change), and DC resistances were measured after charging the cells into a full-charge state (SOC 100%) as an internal maximum energy state and storing them at a high temperature of 60 °C for 60 days to calculate DCIR increase rates (%), and the results are shown in Table 3.

Equation 3

$$\text{DCIR increase rate} = (\text{DCIR after 60 days} / \text{initial DCIR}) * 100$$

Table 2

| | Capacity retention rate @60 °C, 60D(%) | Capacity recovery rate @60 °C, 60D(%) | Initial resistance @25°C (mΩ) | DC-IR @60 °C, 60D (mΩ) | DC-IR change rate (%) |
|---|---|---|---|---|---|
| Example 1 | 77.7 | 81.0 | 21.24 | 28.93 | 136.2 |
| Example 2 | 84.0 | 86.7 | 22.01 | 29.34 | 133.3 |
| Example 3 | 87.5 | 89.5 | 22.38 | 28.91 | 129.2 |
| Example 4 | 85.9 | 88.8 | 22.69 | 29.84 | 131.5 |
| Example 5 | 84.0 | 86.4 | 23.40 | 30.89 | 132.0 |
| Example 6 | 75.1 | 79.5 | 21.10 | 28.97 | 137.3 |
| Example 7 | 79.0 | 81.2 | 21.57 | 29.46 | 136.6 |

(continued)

| | Capacity retention rate @60 °C, 60D(%) | Capacity recovery rate @60 °C, 60D(%) | Initial resistance @25°C (mΩ) | DC-IR @60 °C, 60D (mΩ) | DC-IR change rate (%) |
|---|---|---|---|---|---|
| Example 8 | 83.8 | 84.4 | 22.54 | 30.59 | 135.7 |
| Example 9 | 84.0 | 85.0 | 22.44 | 30.47 | 135.8 |
| Example 10 | 79.8 | 82.3 | 23.00 | 31.53 | 137.1 |
| Example 11 | 89.5 | 93.5 | 19.20 | 24.80 | 129.2 |
| Comparative Example 1 | 61.0 | 73.7 | 21.00 | 31.94 | 152.1 |
| Comparative Example 2 | 65.9 | 74.0 | 22.90 | 33.16 | 144.8 |

Referring to Table 2, the rechargeable lithium battery cells of Examples 1 to 11, compared with the cells of Comparative Examples 1 and 2, each exhibited improved capacity retention and an improved capacity recovery rate during the high-temperature storage but were each suppressed or reduced from an increase in resistance.

**Summary**

Referring to Tables 1 and 2, Examples 1 to 11 each utilizing a compound having a structure in which two acid anhydrides were linked through an aliphatic or aromatic ring (Chemical Formula 1) as an electrolyte additive, compared with Comparative Example 1 utilizing no additive, showed improved storage characteristics at a high temperature, as well as room temperature charge and discharge characteristics of the rechargeable lithium battery cells. For example, Examples 1 to 10 each utilizing a cobalt-free positive electrode active material exhibited evident effects.

In contrast, in Comparative Example 2 utilizing a linear acid anhydride-based compound as an electrolyte additive and Comparative Example 3 utilizing an aliphatic cyclic acid anhydride-based compound as an electrolyte additive, the additives were reduced and decomposed on the negative electrode surface and formed an unstable film, deteriorating a cycle-life as well as increasing resistance of the rechargeable lithium battery cells. This phenomenon became more significant (e.g., noticeable), if stored at a high temperature.

Reference Numerals

100: rechargeable lithium battery
10: positive electrode
20: negative electrode
30: separator
40: electrode assembly
50: case
60: sealing member
70: electrode tab

**Claims**

**1.** An additive for an electrolyte of a rechargeable lithium battery (100), the electrolyte additive being represented by Chemical Formula 1:

Chemical Formula 1

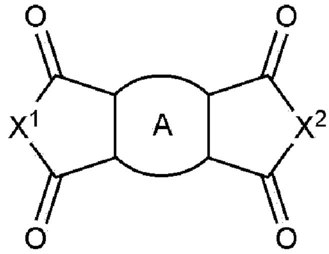

in Chemical Formula 1,

A is a substituted or unsubstituted C3 to C10 aliphatic ring, or a substituted or unsubstituted C6 to C20 aromatic ring; and
$X^1$ and $X^2$ are each independently O or S,
wherein "substituted" refers to replacement of at least one hydrogen of a compound by a substituent selected from among a halogen atom, a hydroxy group, a C1 to C20 alkoxy group, a nitro group, a cyano group, amine group, an imino group, an azido group, an amidino group, a hydrazino group, a hydrazono group, a carbonyl group, a carbamyl group, a thiol group, an ester group, an ether group, a carboxyl group or a salt thereof, a sulfonic acid group or a salt thereof, a phosphoric acid or a salt thereof, a C1 to C20 alkyl group, a C2 to C20 alkenyl group, a C2 to C20 alkynyl group, a C6 to C20 aryl group, a C3 to C20 cycloalkyl group, a C3 to C20 cycloalkenyl group, a C3 to C20 cycloalkynyl group, a C2 to C20 heterocycloalkyl group, a C2 to C20 heterocycloalkenyl group, a C2 to C20 heterocycloalkynyl group, and a C3 to C20 heteroaryl group.

**2.** The additive as claimed in claim 1, wherein A is a cyclohexane ring or a benzene ring.

**3.** The additive as claimed in claim 1 or 2, wherein $X^1$ and $X^2$ are each O.

**4.** The additive as claimed in claim 1, wherein the electrolyte additive is represented by Chemical Formula 1-1 or Chemical Formula 1-2:

Chemical Formula 1-1

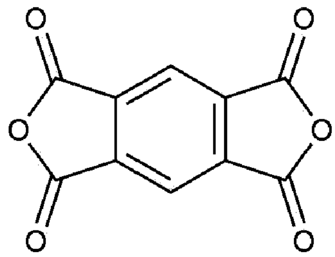

Chemical Formula 1-2

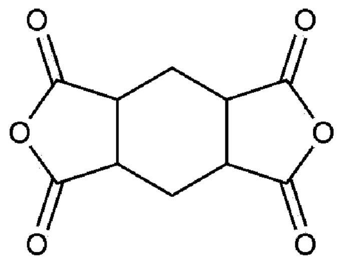

**5.** An electrolyte comprising:

a non-aqueous organic solvent;

a lithium salt; and
the additive according to claim 1.

6. The electrolyte as claimed in claim 5, wherein the electrolyte comprises the additive in an amount of0.05 wt% to3.0 wt%, based on 100 wt% of the electrolyte.

7. The electrolyte as claimed in claim 5 or 6, wherein the non-aqueous organic solvent comprises at least one selected from among a carbonate-based solvent, an ester-based solvent, an ether-based solvent, a ketone-based solvent, an alcohol-based solvent, and a combination thereof.

8. The electrolyte as claimed in any one of claims 5 to 7 wherein the non-aqueous organic solvent comprises ethylene carbonate (EC), ethylmethyl carbonate (EMC), and dimethyl carbonate (DMC).

9. The electrolyte as claimed in claim 8, wherein a volume ratio of ethylene carbonate (EC), ethylmethyl carbonate (EMC), and dimethyl carbonate (DMC) is 2:4:4.

10. A rechargeable lithium battery (100), the rechargeable lithium battery (100) comprising:

a positive electrode (10);
a negative electrode (20); and
the electrolyte according to claim 5.

11. The rechargeable lithium battery (100) as claimed in claim 10, wherein the positive electrode comprises a positive electrode active material comprising a lithium nickel-based composite oxide.

12. The rechargeable lithium battery (100) as claimed in claim 11, wherein the lithium nickel-based composite oxide is represented by Chemical Formula 11:

Chemical Formula 11 $Li_{a1}Ni_{x1}M^1y_1M^2{}_{z1}O_{2-b1}X_{b1}$

in Chemical Formula 11,

$0.9 \leq a1 \leq 1.2$, $0.6 \leq x1 \leq 1$, $0 \leq y1 \leq 0.3$, $0 \leq z1 \leq 0.3$, $0.9 \leq x1+y1+z1 \leq 1.1$, and $0 \leq b1 \leq 0.1$;
$M^1$ and $M^2$ are each independently at least one element selected from among Al, B, Ba, Ca, Ce, Co, Cr, Cu, Fe, Mg, Mn, Mo, Nb, Si, Sr, Ti, V, W, and Zr; and
X is at least one element selected from among F, P, and S.

13. The rechargeable lithium battery (100) as claimed in any one of claims 10 to 12, wherein the rechargeable lithium battery comprises a separator (30) between the positive electrode (10) and the negative electrode (20).

14. A method of manufacturing a rechargeable lithium battery (100), the method comprising:

preparing an electrolyte according to claim 5;
preparing a positive electrode (10);
preparing a negative electrode (20);
preparing an electrode assembly comprising the positive electrode (10), the negative electrode (20), and a separator (30); and
injecting the electrolyte into the electrode assembly.

15. The method as claimed in claim 14, wherein the non-aqueous organic solvent comprises at least one selected from among a carbonate-based solvent, an ester-based solvent, an ether-based solvent, a ketone-based solvent, an alcohol-based solvent, and a combination thereof.

FIG. 1

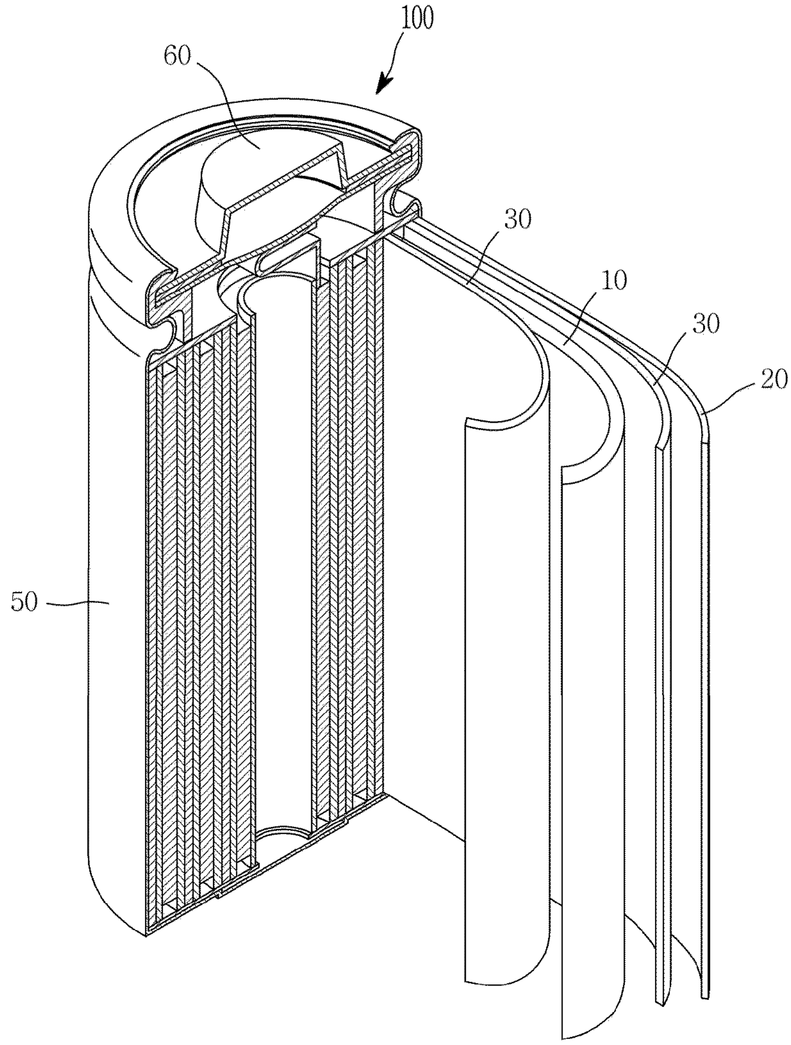

FIG. 2

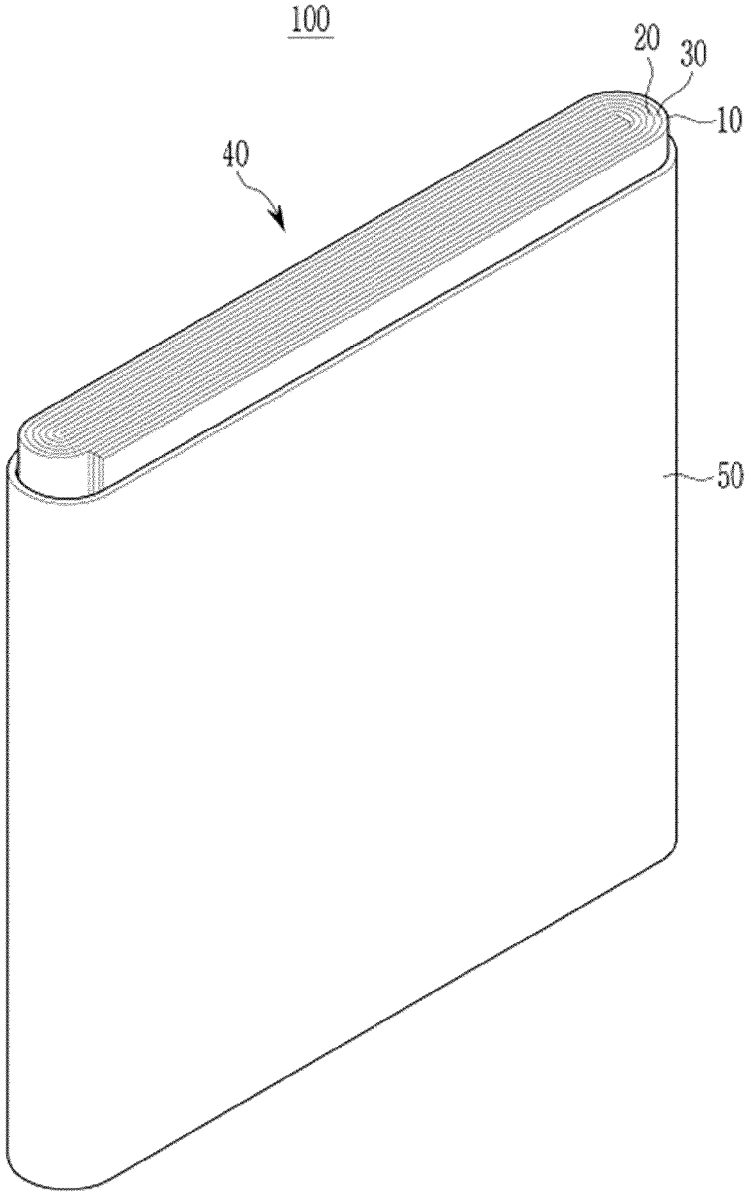

FIG. 3

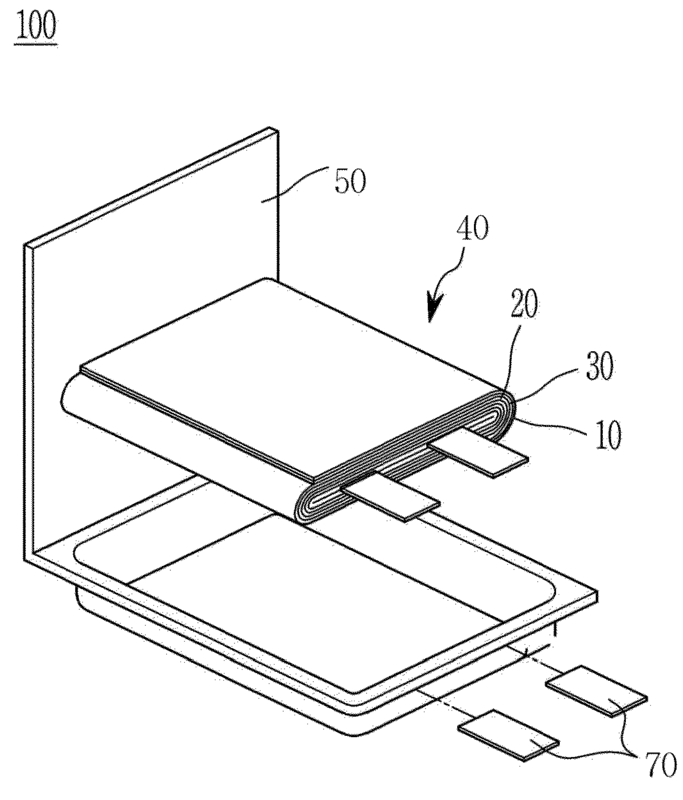

Europäisches Patentamt
European Patent Office
Office européen des brevets

# PARTIAL EUROPEAN SEARCH REPORT

under Rule 62a and/or 63 of the European Patent Convention. This report shall be considered, for the purposes of subsequent proceedings, as the European search report

Application Number
EP 24 17 9763

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | CN 104 466 249 A (XUE LI)<br>25 March 2015 (2015-03-25)<br>* claims 1-5 *<br>----- | 5-8,<br>10-15<br>9 | INV.<br>H01M4/525<br>H01M10/0567 |
| X<br><br><br>A | CN 107 887 647 A (GUANGZHOU TINCI MATERIALS TECH CO LTD)<br>6 April 2018 (2018-04-06)<br>* claims 1-8 *<br>* example 1 *<br>----- | 5-8,<br>10-15<br><br>9 | |
| | | | TECHNICAL FIELDS SEARCHED (IPC) |
| | | | H01M |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

2

| Place of search | Date of completion of the search | Examiner |
|---|---|---|
| The Hague | 22 January 2025 | Domínguez Gutiérrez |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

Europäisches Patentamt
European Patent Office
Office européen des brevets

# INCOMPLETE SEARCH SHEET C

Application Number
EP 24 17 9763

Claim(s) completely searchable:
-

Claim(s) searched incompletely:
5-15

Claim(s) not searched:
1-4

Reason for the limitation of the search:

1 The Applicant had been invited to file a statement indicating the subject-matter to be searched within the time limit indicated in the previous communication (Rule 63(1) EPC) because the claims were not considered to meet the relevant requirements of Art. 84 EPC to such an extent that no search of claims 1-4, and only a partial search of the claims 5-15 was possible. The Applicant replied with an alternative set of claims on which the search should be based, and arguments received on 29/November/2024.
The reply includes arguments a request for a search in favour of the said set of claims.

2. A search is expected for claim 4, which was previously considered unclear as a basis for the search. The Applicant considers that a claim based solely on these compounds is clear and supported.
As mentioned in the invitation, only pyromellitic dianhydride and its derivatives encompass applications in epoxy resins, fuel cells, supercapacitors, corrosion-resistant materials, etc.
Pyromellitic dianhydride itself has been known for over a century and began to be widely used and commercialized a few decades later. Since such a compound is notoriously known, no meaningful search is possible (Rule 63 EPC and Guidelines B-VIII, 3).
Protection sought for notoriously known compounds that lack specificity regarding any technical property fails to comply with the fundamental requirements of clarity under Art. 84 EPC.

3. Regarding the wording submitted for claims 5-15, this one is seen as sufficiently supported and clarifying.
The search for claims 5-15 has therefore been based on said wording.
All claims referring to it are accordingly affected. These claims are however not considered to be the claims on file, according to R. 173(1) EPC. This opinion has been drafted in respect of the numbering of the originally filed claims.

4. The Applicant's attention is drawn to the fact that the application will be further prosecuted on the basis of the subject-matter for which a search has been carried out, and that the claims should be limited to said subject-matter at a later stage of the proceedings (R. 63 EPC).
Amendments may relate neither to subject-matter that was excluded from the search following the invitation under Rule 63(1) EPC, nor to non-searched subject-matter that does not combine with the originally claimed invention or group of inventions to form a single general inventive concept (Rule 137(5) EPC).

# ANNEX TO THE EUROPEAN SEARCH REPORT ON EUROPEAN PATENT APPLICATION NO.

EP 24 17 9763

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2025

| Patent document cited in search report | Publication date | Patent family member(s) | Publication date |
|---|---|---|---|
| CN 104466249 A | 25-03-2015 | NONE | |
| CN 107887647 A | 06-04-2018 | NONE | |

EPO FORM P0459

For more details about this annex : see Official Journal of the European Patent Office, No. 12/82